# EUROPÄISCHE PATENTSCHRIFT

(11) **EP 1 062 943 B1**
(45) Veröffentlichungstag und Bekanntmachung des Hinweises auf die Patenterteilung: **09.01.2008**
(21) Anmeldenummer: 00110191.4
(22) Anmeldetag: 15.05.2000
(51) Int. Cl.: A61K 8/19, A61K 8/49, A61Q 1/02, A61Q 17/04

(54) **Kosmetische oder dermatologische Lichtschutzzubereitungen mit einem Gehalt an Bornitrid und UV-Filtersubstanzen, welche das Strukturmotiv des Triazins aufweisen**
Cosmetic or dermatologic sunscreen compositions containing boron nitride and UV filters having triazine groups
Compositions photoprotectices cosmétiques ou dermatologiques contenant du nitrure de bore et des filtres UV avec des groupements triazine

(30) Priorität: 22.05.1999 DE 19923667
(43) Veröffentlichungstag der Anmeldung: 27.12.2000
(73) Patentinhaber: Beiersdorf Aktiengesellschaft, 20245 Hamburg (DE)
(72) Erfinder: Gers-Barlag, Heinrich, Dr., 25459 Kummerfeld (DE); Dörschner, Albrecht, 20146 Hamburg (DE); Schomann, Ariane, 22453 Hamburg (DE); Müller, Anja, 23843 Rümpel (DE)

(56) Entgegenhaltungen:
- EP-A- 0 087 098
- EP-A- 0 775 698
- EP-A- 0 987 004
- EP-A- 0 987 007
- EP-A- 0 987 008
- EP-A- 1 013 262
- EP-A- 1 013 263
- WO-A-00/07549
- INTROINI C: "NITRURO DI BORO NUOVO INGREDIENTE NEI PRODOTTI PER IL CORPO E NEI SOLARI" COSMETICS AND TOILETRIES. ED. ITALIANA, COSMEO EDITRICE, MILANO, IT, Bd. 18, Nr. 2, 1997, Seiten 71,73,75-76, XP000853904 ISSN: 1121-1377

## Beschreibung

Die vorliegende Erfindung betrifft Lichtschutzformulierungen, insbesondere kosmetische und dermatologische Lichtschutzmittel.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letzlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Epidermis ist ein stratifiziertes Gewebe, in dem die äußere Schicht, die Hornschicht (Stratum corneum), den für die Barrierefunktion bedeutenden Teil darstellt. Das heute in der Fachwelt anerkannte Hautmodell von Elias (P. M. Elias, Structure and Function of the Stratum Corneum Permeability Barrier, Drug Dev. Res. 13, 1988, 97-105) beschreibt die Hornschicht als Zwei-Komponenten-System, ähnlich einer Ziegelsteinmauer (Ziegelstein-Mörtel-Modell). In diesem Modell entsprechen die Hornzellen (Korneozyten) den Ziegelsteinen, die komplex zusammengesetzte Lipidmembran in den Interzellularräumen entspricht dem Mörtel. Dieses System stellt im wesentlichen eine physikalische Barriere gegen hydrophile Substanzen dar, kann aber aufgrund seiner engen und mehrschichtigen Struktur gleichermaßen auch von lipophilen Substanzen nur schwer passiert werden.

Die schadigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubsubstanzen zur Verfügung zu haben, da auch dessen Strahlen Schäden hervorrufen können. Man hat lange Zeit fälschlicherweise angenommen, daß die langwellige UV-A-Strahlung mit einer Wellenlänge zwischen 320 nm und 400 nm nur eine vernachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u.a. erwiesen, daß selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z.B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Vorbeugender Schutz gegen UV-A-Strahlen, beispielsweise durch Auftrag von Lichtschutzfiltersubstanzen in Form einer kosmetischen oder dermatologischen Formulierung auf die Haut, ist daher von grundsätzlicher Wichtigkeit.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen. Für die Dosierung der Substanzen in den fertigen Formulierungen können die Extinktionswerte allenfalls eine Orientierungshilfe bieten, denn durch Wechselwirkungen mit Inhaltsstoffen der Haut oder der Hautoberfläche selbst können Unwägbarkeiten auftreten. Ferner ist in der Regel schwierig vorab abzuschätzen, wie gleichmäßig und in welcher Schichtdicke die Filtersubstanz in und auf der Hornschicht der Haut verteilt ist.

Zur Prüfung der UV-A-Schutzleistung wird üblicherweise die IPD-Methode verwendet (IPD=immediate pigment darkening). Hierbei wird - ähnlich der Bestimmung des Lichtschutzfaktors - ein Wert ermittelt, der angibt, um wieviel länger die mit dem Lichtschutzmittel geschützte Haut mit UV-A-Strahlung bestrahlt werden kann, bis die gleiche Pigmentierung auftritt wie bei der ungeschützten Haut.

Eine andere, europaweit etablierte Prüfungsmethode ist der Australische Standard AS/NZS 2604:1997. Dabei wird die Absorption der Zubereitung im UV-A-Bereich gemessen. Um den Standard zu erfüllen, muß die Zubereitung mindestens 90 % der UV-A-Strahlung im Bereich 320 -360 nm absorbieren.

Da Lichtschutzfiltersubstanzen in der Regel kostspielig sind und da manche Lichtschutzfiltersubstanzen zudem schwierig in höheren Konzentrationen in kosmetische oder dermatologische Zubereitungen einzuarbeiten sind, war es eine Aufgabe der Erfindung, auf einfache und preiswerte Weise zu Zubereitungen zu gelangen, welche bei ungewöhnlich niedrigen Konzentrationen an herkömmlichen UV-A-Lichtschutzfiltersubstanzen dennoch eine akzeptable oder sogar hohe UV-A-Schutzleistung erreichen.

Eine weitere Aufgabe der Erfindung war es, auf einfache und preiswerte Weise zu Zubereitungen zu gelangen, welche bei ungewöhnlich niedrigen Konzentrationen an herkömmlichen UV-B-Lichtschutzfiltersubstanzen dennoch akzeptable oder sogar hohe LSF-Werte erreichen.

Ferner war Aufgabe der Erfindung durch ungewöhnlich niedrige Konzentrationen an UV-A- und UV-B-Filtersubstanzen und/oder Breitbandfiltern eine dennoch akzeptable oder sogar hohe Breitbandwirkung zu erreichen.

Die UV-Strahlung kann auch zu photochemischen Reaktionen führen, wobei dann die photochemischen Reaktionsprodukte in den Hautmetabolismus eingreifen. Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Um diesen Reaktionen vorzubeugen, können den kosmetischen bzw. dermatologischen Formulierungen zusätzlich Antioxidantien und/oder Radikalfänger einverleibt werden.

Vorteilhafte Lichtschutzfilter, die sich durch das Strukturmotiv des Triazins auszeichnen, beruhen in der Regel auf der Basis von 1,3,5-Triazinen (synonym: *s*-Triazine) 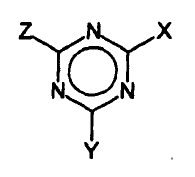 wobei die Substituenden X, Y und Z als zusätzliche Chromophore im weiteren Sinne der UV-Absorption zu verstehen sind.

Solche Triazinderivate werden beispielsweise in der EP-A 0 087 098 beschrieben. Die dort beschriebenen Verbindungen tragen die allgemeine Struktur 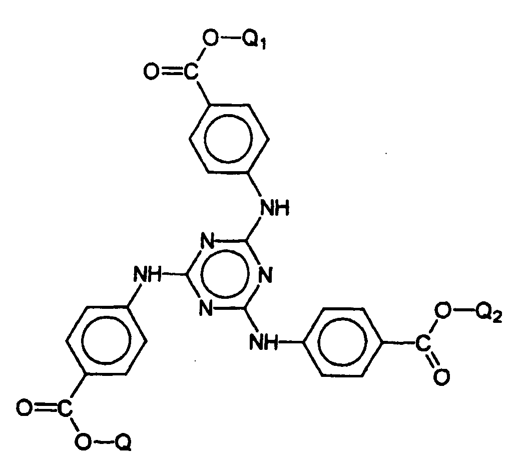 wobei Q, Q₁ und Q₂ gleich oder verschieden sein können und für Wasserstoff, Alkalimetall, ggf. durch organische Reste substituiertes Ammonium, C₁₋₂₀ -Alkyl oder einen Polyoxyethylenrest mit 1 - 10 Ethylenoxideinheiten, deren endständige OH-Gruppe durch einen Alkohol mit 1 bis 3 C-Atomen verethert sein kann, stehen.

Ein ganz besonders vorteilhafter UVB-Filter aus dieser Verbindungsgruppe ist der 4,4',4''-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin.

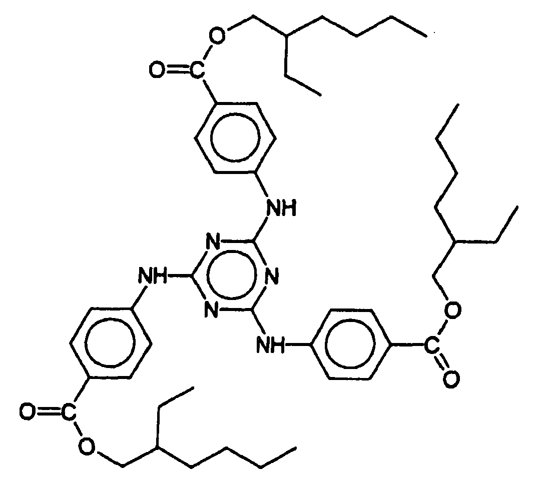

Diese UVB-Filtersubstanz wird von der BASF Aktiengesellschaft unter der Warenbezeichnung UVINUL^{®} T 150 vertrieben und zeichnet sich durch gute UV-Absorptions-eigenschaften aus.

Ein weiterer vorteilhafter UV-Filter ist das Dioctylbutamidotriazon, dessen chemische Struktur durch die Formel 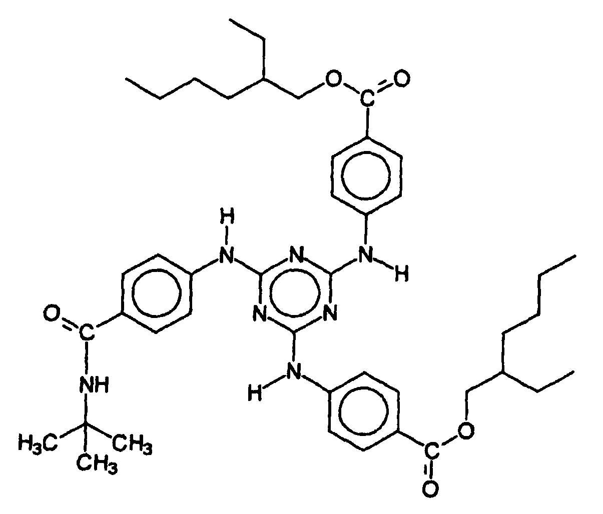 wiedergegeben wird. Diese UV-Filtersubstanz wird von Gesellschaft 3V SIGMA unter der Warenbezeichnung Uvasorb HEB vertrieben und zeichnet sich durch gute UV-Absorptionseigenschaften aus. Ferner zeichnet sich diese Substanz durch die Eigenschaft aus, auf der Haut unmerklich dünne Filme zu bilden, die einesteils erwünscht sein können, andererseits aber auch taktile Nachteile aufweisen.

Es war also eine Aufgabe der vorliegenden Erfindung, den Nachteilen des Standes der Technik abzuhelfen. Ferner war es eine Aufgabe der vorliegenden Erfindung, kosmetische oder dermatologische Lichtschutzzubereitungen zu konzipieren, welche sich durch erhöhte Pflegewirkung auszeichnen.

Bornitrid (BN oder besser B_{∞}N_{∞}) ist ein homogenes Pulver, welches bekanntermaßen die Eigenschaften kosmetischer Pudergrundlagen verbessert. Im folgenden sind die Strukturen der beiden Hauptmodifikationen des Bornitrids schematisch angegeben, wobei auf die Angabe mesomerer Grenzstrukturen (beim hexagonalen α-Bornitrid) sowie der formalen Ladungen (beim kubischen β-Bornitrid) verzichtet wurde.

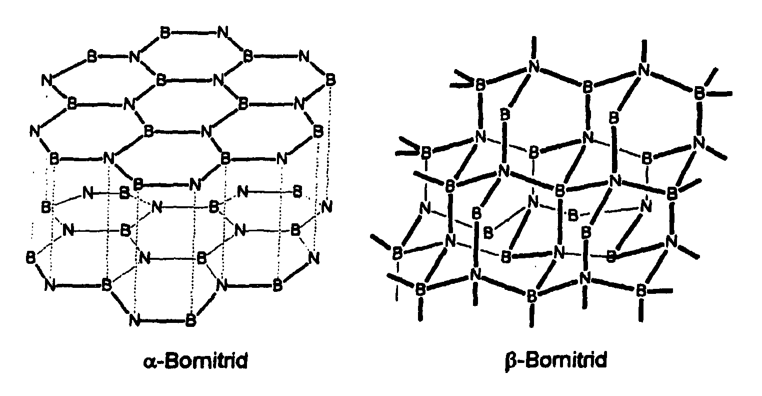

Es war indes überraschend und für den Fachmann nicht vorauszusehen, daß kosmetische oder dermatologische Lichtschutzzubereitungen mit einem Gehalt an Bornitrid und kosmetisch oder dermatologischen UV-Filtersubstanzen, welche das Strukturmotiv des Triazins aufweisen, mit Ausnahme solcher Zubereitungen,
- welche höchstens 0,5 Gew.-% eines oder mehrerer Emulgatoren enthalten, und mit Ausnahme solcher Zubereitungen,
- welche mindestens eine polare Ölkomponente enthalten,
sowie die Verwendung von Bornitrid zur Erhöhung des Lichtschutzfaktors kosmetischer oder dermatologischer Zubereitungen mit einem Gehalt an kosmetisch oder dermatologisch verträglichen UV-Filtersubstanzen, welche das Strukturmotiv des Triazins enthalten, den Nachteilen des Standes der Technik abhelfen würden.

Es war für den Fachmann daher nicht vorauszusehen gewesen, daß die erfindungsgemäßen Wirkstoffkombinationen bzw. kosmetische oder dermatologische Zubereitungen, diese enthaltend
- besser die Bindung von schädlichen Photoprodukten an Lipide, DNS und Proteine verhindern
- besser gegen die Hautalterung wirken
- besser die Haut gegen Photoreaktionen schützen
- besser entzündlichen Reaktionen vorbeugen würde
- besser gegen Gelbfärbung geschützt sein würde,
- zu synergistisch verstärkter Erhöhung des Lichtschutzfaktors und/oder der UV-A-Schutzleistung kosmetischer oder dermatologischer Lichtschutzmittel führen würde
als die Wirkstoffe, Wirkstoffkombinationen und Zubereitungen des Standes der Technik. C. Introini "Nitruro di Boro; Nuovo Ingrediente nei Prodotti per il Corpo e nei Solari"; Cosmetics and Toiletries; Ed. Italiana, Cosmeo Editrice, Milano, IT, Bd. 18, Nr.2, 1997, Seiten 71, 73, 75-76 beschreibt die Verwendung von Bornitrid in kosmetischen Lichtschutzmitteln.

Das α-Bornitrid stellt die erfindungsgemäß bevorzugt eingesetze Modifikation dar. Es ist dabei bevorzugt, das α-Bornitrid zu wählen, insbesondere solches, welches eine mittlere primäre Partikelgröße von 0,1 - 50 µm, bevorzugt 0,1 - 10 µm aufweist.

Erfindungsgemäß enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% Bornitrid

Erfindungsgemäß vorteilhaft können das oder die UV-Filtersubstanzen, welche das Strukturmotiv des Triazins aufweisen gewählt werden aus der Gruppe der nachfolgend näher bezeichneten Substanzen: 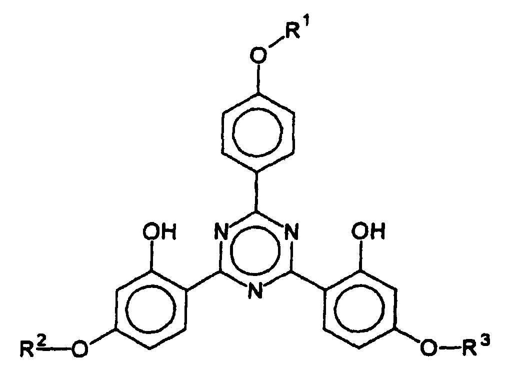 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt ist das 2,4-Bis-{[4-(2-Ethyl-hexyloxy)-2-hydroxy]-phenyl}-6-(4-methoxyphenyl)-1,3,5-triazin, welches durch folgende Struktur gekennzeichnet ist:

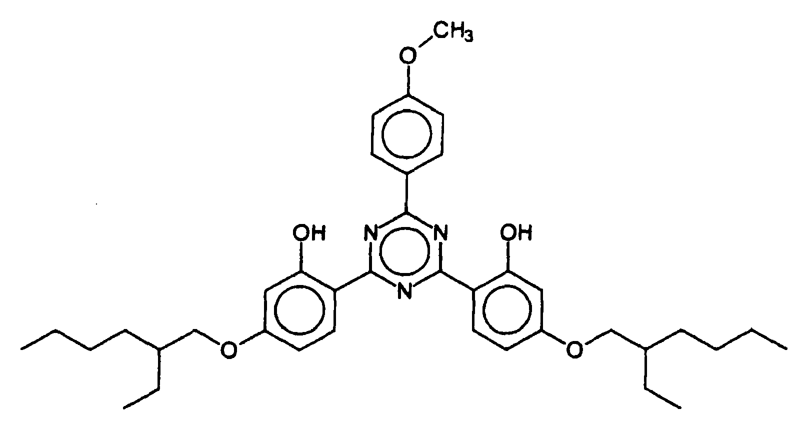

Ein weiterer vorteilhafter UV-Filter ist der 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin.

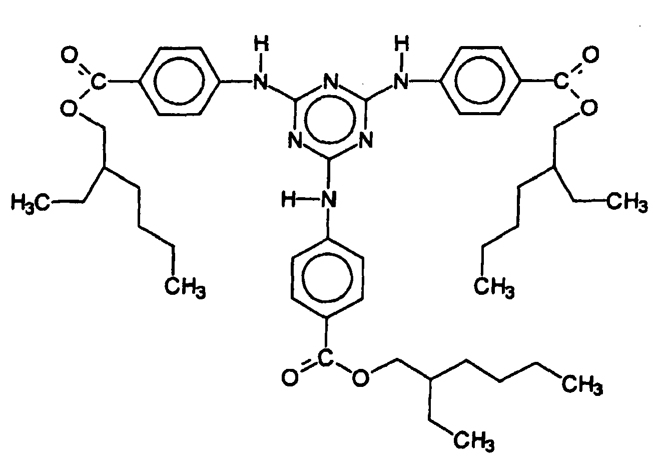

Diese UV-B-Filtersubstanz wird von der BASF Aktiengesellschaft unter der Warenbezeichnung UVINUL^{®} T 150 vertrieben und zeichnet sich durch gute UV-Absorptionseigenschaften aus. Der Hauptnachteil des 4,4'4''-(1,3,5-Triazin-2,4,6-triyltriimino)-tris-benzoësäure-tris(2-ethylhexylesters) ist seine schlechte Löslichkeit in Lipiden. Es bereitete daher in der Vergangenheit gewisse formulierungstechnische Schwierigkeiten, mit Hilfe dieses Filters höhere Lichtschutzfaktoren zu erzielen, was aber durch die Lehre der Erfindung überkommen werden konnte.

Ferner vorteilhaft sind auch UV-Filtersubstanzen beschrieben, welche das Strukturmotiv 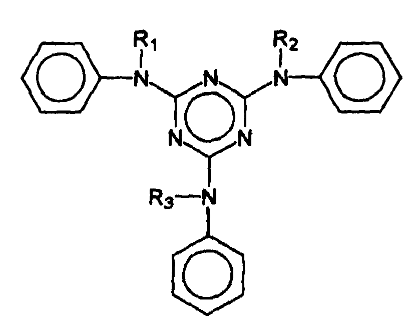 aufweisen, wie die in der Europäischen Offenlegungsschrift 570 838 beschriebenen s-Triazinderivate, deren chemische Struktur durch die generische Formel 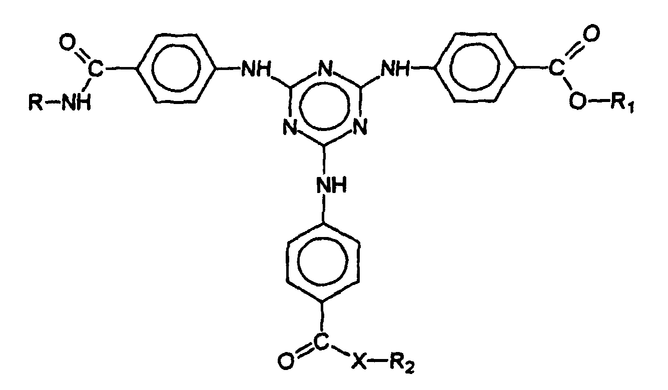 wiedergegeben wird, wobei
- R: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkyl-rest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 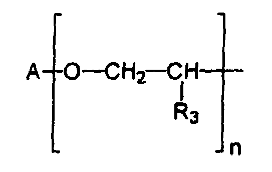 bedeutet, in welcher
- A: einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
- R₃: ein Wasserstoffatom oder eine Methylgruppe darstellt,
- n: eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 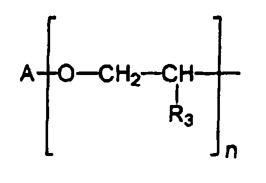 bedeutet, in welcher
- A: einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
- R₃: ein Wasserstoffatom oder eine Methylgruppe darstellt,
- n: eine Zahl von 1 bis 10 darstellt,
- wenn X: ein Sauerstoffatom darstellt.

Ein Beispiel für solche unsymmetrisch substituierte s-Triazine stellt das Dioctylbutylamidotriazon dar, dessen chemische Struktur durch die Formel 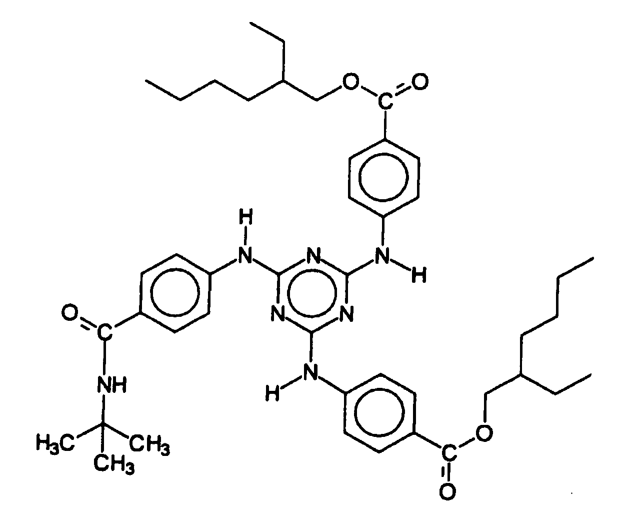 wiedergegeben wird.

Auch andere UV-Filtersubstanzen, deren Einarbeitung in kosmetische oder dermatologische Lichtschutzformulierungen Probleme aufweist, sind bekannt. So werden in der Europäischen Offenlegungsschrift 775 698 Bis-Resorcinyltriazinderivate beschrieben, deren chemische Struktur durch die generische Formel 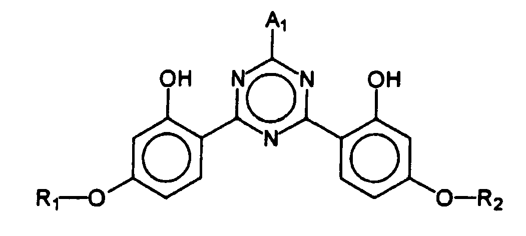 wiedergegeben wird, wobei R₁, R₂ und A₁ verschiedenste organische Reste repräsentieren.

Ferner vorteilhaft im Sinne der vorliegenden Erfindung ist das 2,4-Bis-{[4-(3-sulfonato)-2-hydroxy-propyloxy)-2-hydroxy}-phenyl}-6-(4-methoxyphenyl)-1,3,5-triazin Natriumsalz, welches durch folgende Struktur gekennzeichnet ist:

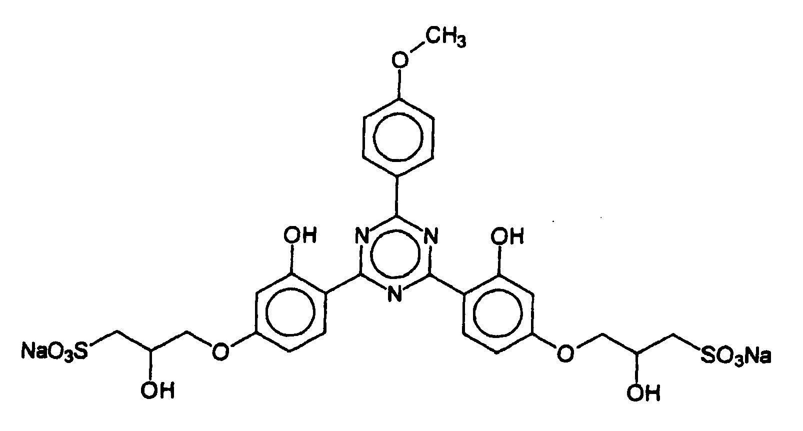

Ferner vorteilhaft ist das 2,4-Bis-{[4-(3-(2-Propyloxy)-2-hydroxy-propyloxy)-2-hydroxy]-phenyl}-6-(4-methoxyphenyl)-1,3,5-triazin, welches durch folgende Struktur gekennzeichnet ist:

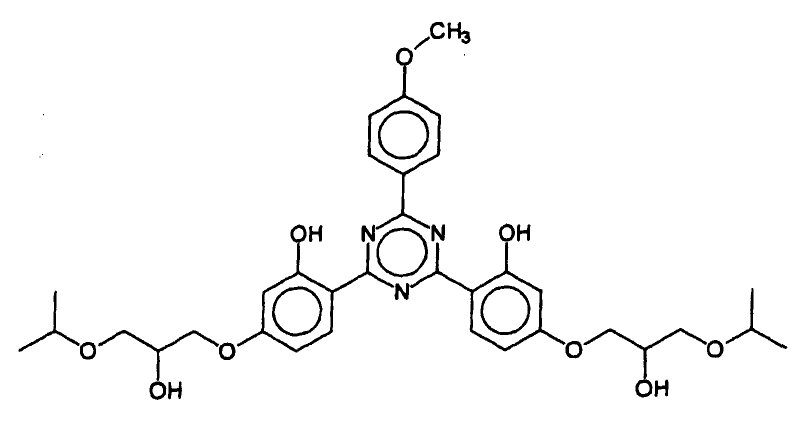

Ferner vorteilhaft ist das 2,4-Bis-{[4-(2-ethyl-hexyloxy)-2-hydroxy]-phenyl}-6-[4-(2-methoxyethyl-carboxyl)-phenylamino]-1,3,5-triazin, welches durch folgende Struktur gekennzeichnet ist:

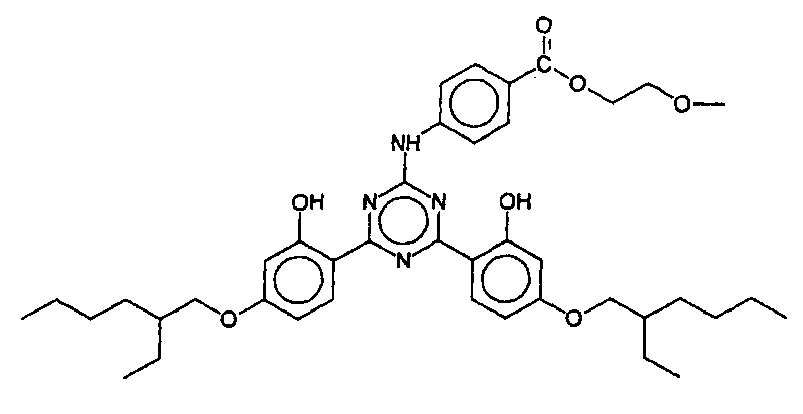

Ferner vorteilhaft ist das 2,4-Bis-{[4-(3-(2-propyloxy)-2-hydroxy-propyloxy)-2-hydroxy]-phenyl}-6-[4-(2-ethylcarboxyl)-phenylamino]-1,3,5-triazin, welches durch folgende Struktur gekennzeichnet ist:

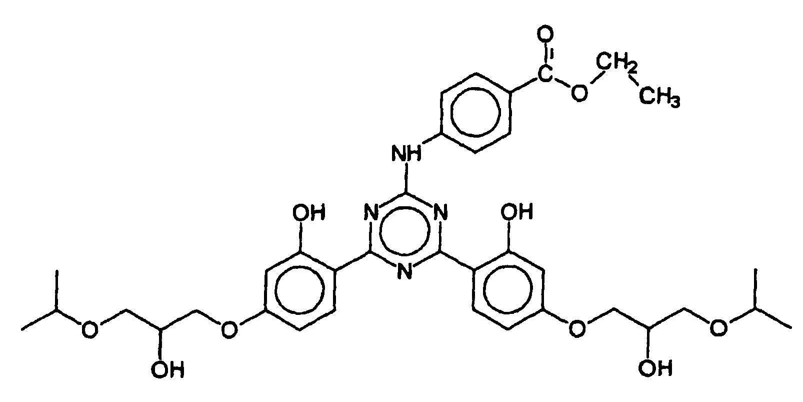

Ferner vorteilhaft ist das 2,4-Bis-{[4-(2-ethyl-hexyloxy)-2-hydroxy]-phenyl}-6-(1-methylpyrrol-2-yl)-1,3,5-triazin, welches durch folgende Struktur gekennzeichnet ist:

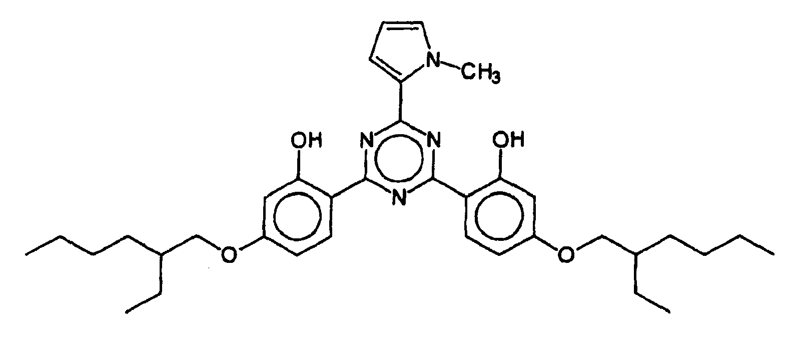

Ferner vorteilhaft ist das 2,4-Bis-{[4-tris(trimethylsiloxy-silylpropyloxy)-2-hydroxy]-phenyl)-6-(4-methoxyphenyl)-1,3,5-triazin, welches durch folgende Struktur gekennzeichnet ist:

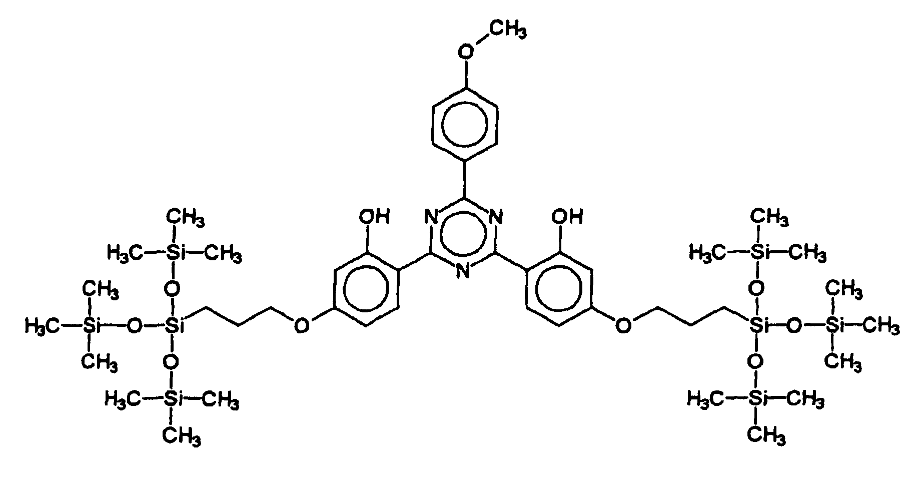

Ferner vorteilhaft ist das 2,4-Bis-{[4-(2"-methylpropenyloxy)-2-hydroxy]-phenyl}-6-(4-methoxyphenyl)-1,3,5-triazin, welches durch folgende Struktur gekennzeichnet ist:

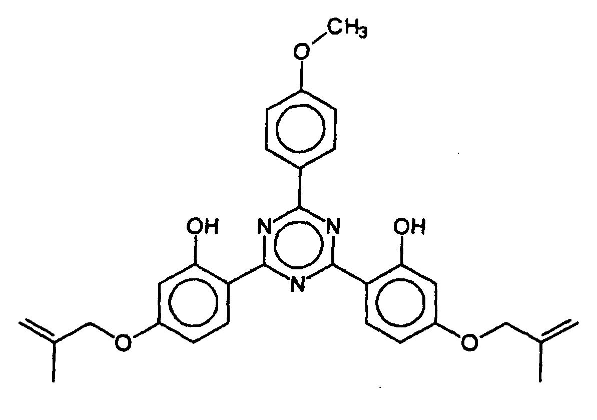

Ferner vorteilhaft ist das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin, welches durch folgende Struktur gekennzeichnet ist:

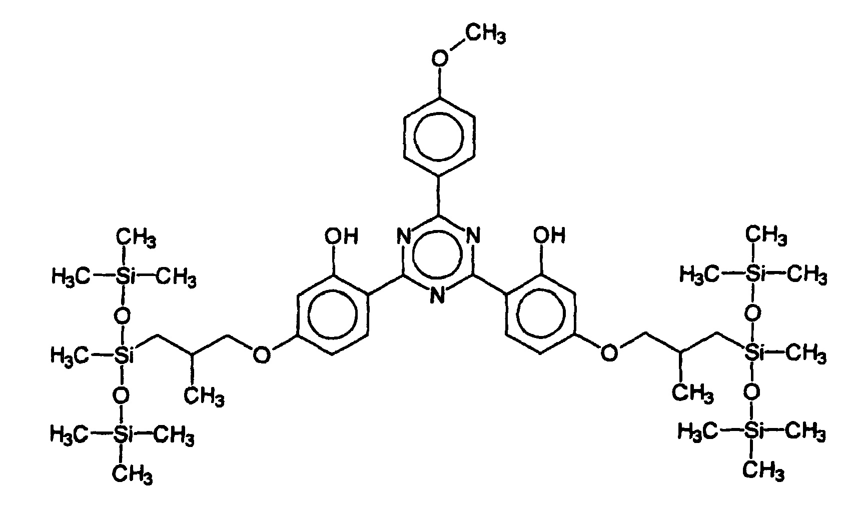

Lichtschutzzubereitungen gemäß der Erfindung sind besonders vorteilhaft, wenn sie 0,1 bis 20 Gew.-%, vorteilhaft 0,5 bis 10 Gew.-%, an UV-Filtersubstanzen, welche das Strukturmotiv des Triazins aufweisen enthalten.

Zusätzliche, ebenfalls vorteilhafte UV-Filtersubstanzen sind die Phenylen-1,4-bis-(2-benzimidazyl)-3,3'-5,5'-tetrasulfonsäure: 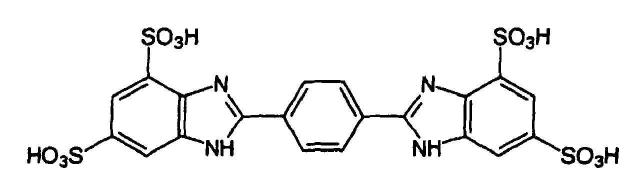 und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz: 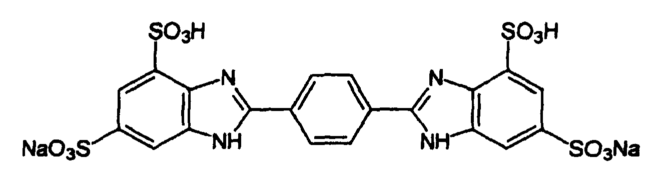 sowie das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

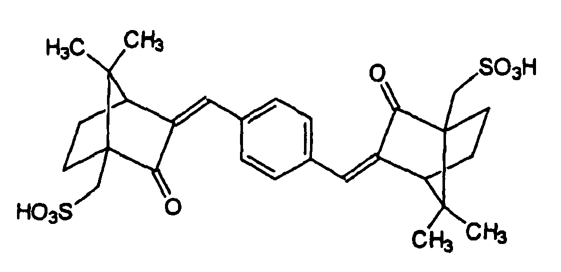

Die Gesamtmenge an wasserlöslichen UV-Filtersubstanzen in den fertigen kosmetischen oder dermatologischen Zubereitungen, beispielsweise an 2-Phenylbenzimidazol-5-sulfonsäure bzw. deren Salzen und/oder 2-Hydroxy-4-methoxybenzophenon-5-sulfonsaure bzw. deren Salzen und/oder 4-(2-Oxo-3-bornylidenmethyl)benzolsulfonsäure bzw. deren Salzen und/oder 2-Methyl-5-(2-oxo-3-bornylidenmethyl)-benzolsulfonsäure bzw. deren Salzen und/oder Benzol-1,4-di(2-oxo-3-bornylidenmethyl)-10-sulfonsäure bzw. deren Salzen, wird vorteilhaft aus dem Bereich von 0,1 - 10,0 Gew.-%, bevorzugt 0,5 - 6,0 Gew.-% gewählt, bezogen auf das Gesamtgewicht der Zubereitungen, falls die Gegenwart dieser Substanzen erwünscht ist.

Weitere vorteilhafte UV-A-Filtersubstanzen im Sinne der vorliegenden Erfindung sind Dibenzoylmethanderivate, insbesondere 5-Isopropyldibenzoylmethan (CAS-Nr. 63250-25-9), welches sich durch die Struktur 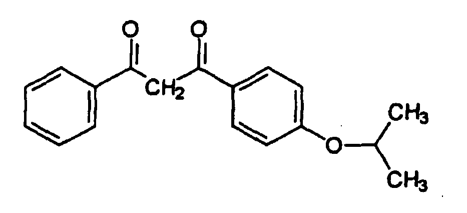 auszeichnet und von Merck unter der Marke Eusolex^{®} 8020 verkauft wird, und/oder das 4-(tert.-Butyl)-4'-methoxydibenzoylmethan (CAS-Nr. 70356-09-1), welches sich durch die Struktur 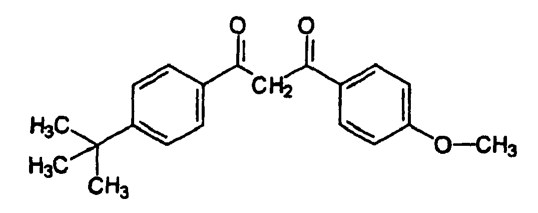 auszeichnet und von Givaudan unter der Marke Parsol ^{®} 1789 verkauft wird.

Weitere im Sinne der vorliegenden Erfindung vorteilhaft einzusetzende Lichtschutzfiltersubstanzen weisen das Strukturelement des Methylidencamphers auf, so beispielsweise der 4-Methylbenzylidencampher, welcher sich durch die Struktur 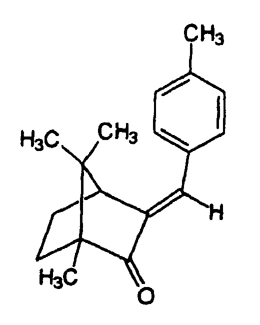 auszeichnet und von Merck unter der Marke Eusolex^{®} 6300 verkauft wird, sowie der Benzylidencampher, welcher sich durch die Struktur 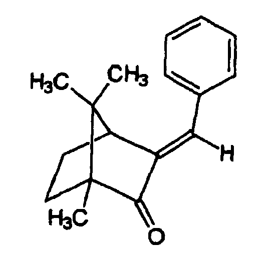 auszeichnet und von der Gesellschaft Induchem unter der Marke Unisol^{®} S22 verkauft wird.

Weitere im Sinne der vorliegenden Erfindung vorteilhaft einzusetzende Lichtschutzfiltersubstanzen weisen das Strukturelement des Benzophenons auf:

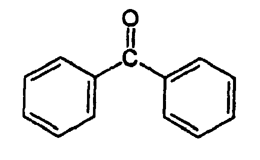

Vorteilhaft können erfindungsgemäß die Substanzen, die das Strukturmotiv des Benzophenons enthalten (in dieser Schrift auch als "Benzophenone" bezeichnet) gewählt werden aus der Gruppe der folgenden Substanzen 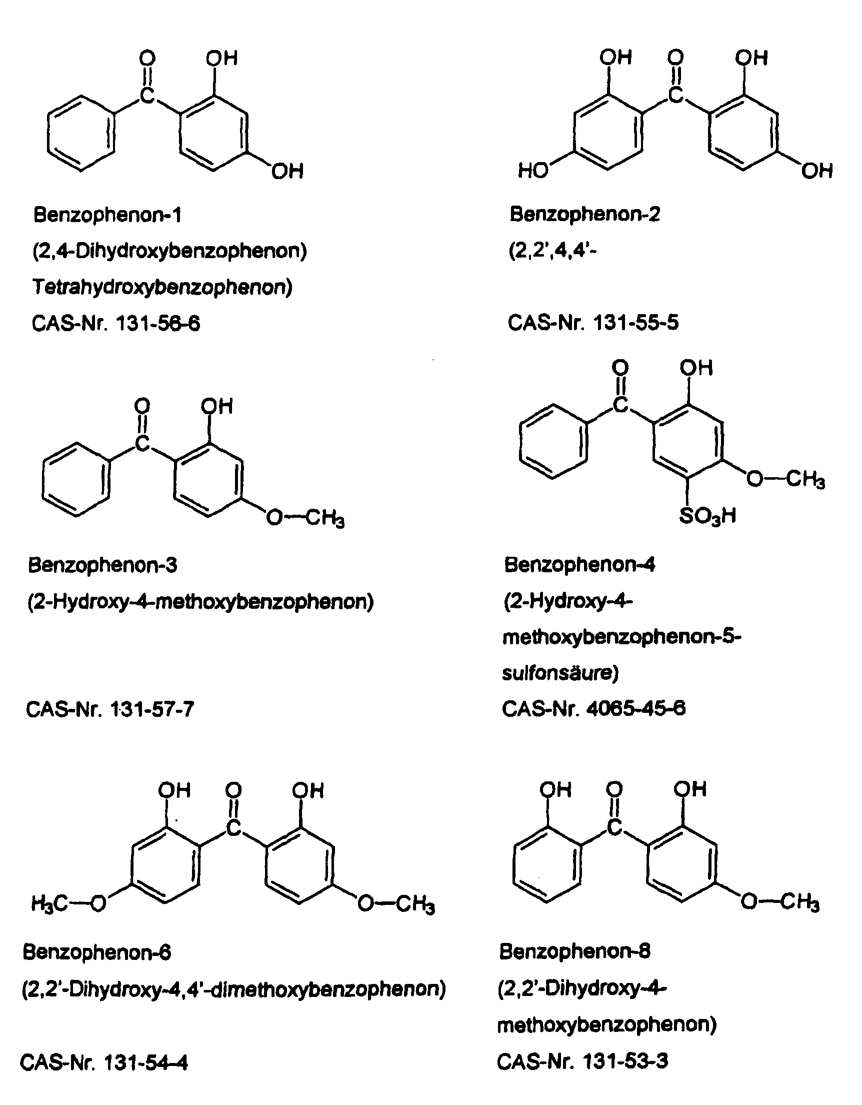 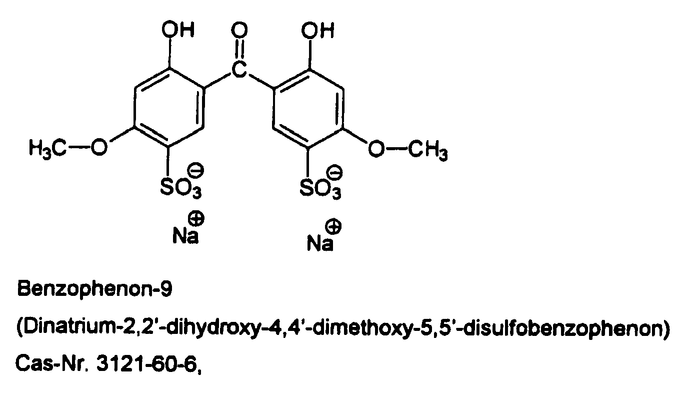 ferner Benzophenon-11, CAS-Nr. 1341-54-4.

Bevorzugtes Benzophenon ist Benzophenon-3, welches beispielsweise von Merck unter der Warenbezeichnung Eusolex^{®} 4360 verkauft wird.

Von den Dibenzoylmethanderivaten werden vorteilhaft verwendet:

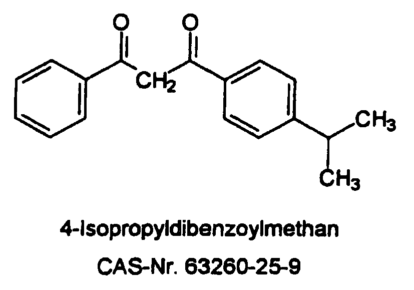

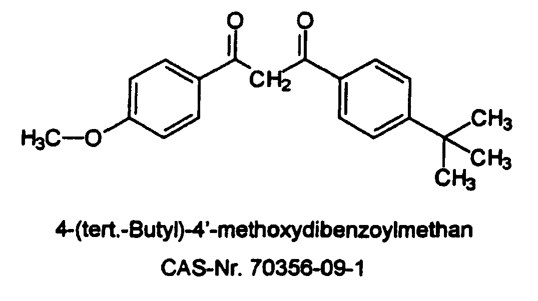

Weitere im Sinne der vorliegenden Erfindung vorteilhaft einzusetzende Lichtschutzfiltersubstanzen sind Salicylsäurederivate wie

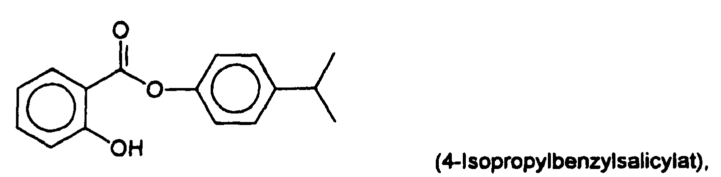

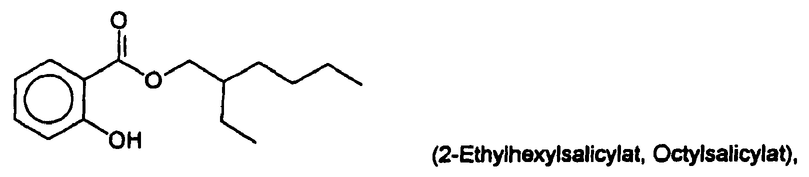

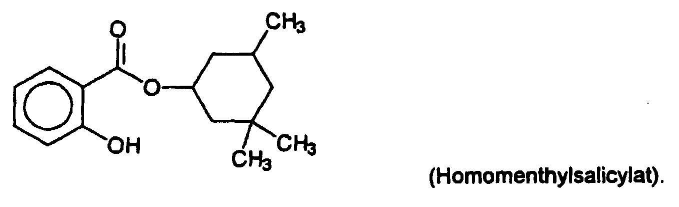

Weiterere im Sinne der vorliegenden Erfindung vorteilhafte Lichtschutzfiltersubstanz sind Zimtsäureester, beispielsweise das 2-Ethylhexyl-p-methoxy-cinnamat (4-Methoxyzimtsäure-2'ethylhexylhester), welches von Givaudan unter der Bezeichnung Parsol^{®} MCX erhältlich ist und sich durch folgende Struktur auszeichnet:

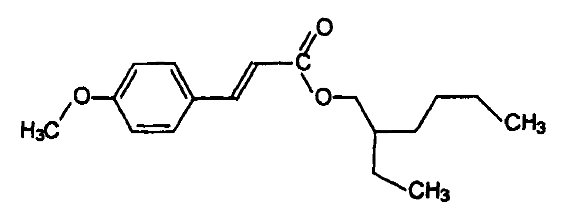

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Noch ein weiterer vorteilhafter UV-Filter ist das 2,2'-Methylen-bis-(6-(2H-benzotriazol-2-yl)4-(1,1,3,3-tetramethylbutyl)-phenol), welches durch die chemische Strukturformel 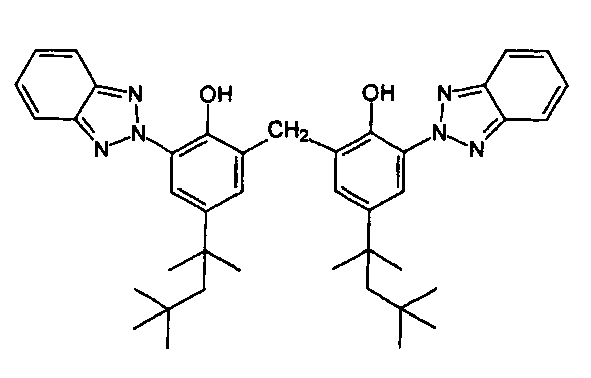 gekennzeichnet ist.

Die gemäß der Erfindung einsetzbaren UV-B-Filter können öllöslich oder wasserlöslich sein. Vorteilhafte öllösliche UV-B-Filtersubstanzen sind z.B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethyl-amino)benzoesäureamylester;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'methy-benzophenon, 2,2'-Dihydroxy-4-methoxybenzophenon;

Im Sinne der Erfindung vorteilhafte wasserlösliche UV-B-Filtersubstanzen sind z.B.:
- Salze der 2-Phenylbenzimidazol-5-sulfonsäure, wie ihr Natrium-, Kalium- oder ihr Triethanolammonium-Salz, sowie die Sulfonsäure selbst;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und deren Salze.

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Insbesondere war erstaunlich, daß Bornitrid im Sinne der vorliegenden Erfindung zu einer Steigerung des Lichtschutzfaktors führen würde, wenn wenigstens einer der UV-Filter in den Lichtschutzzubereitungen gemäß der vorliegenden Erfindung aus der Gruppe der Triazinderivate gewählt wird, insbesondere gewählt aus der Gruppe
- 4,4',4''-(1,3,5-Triazin-2,4,6-triyltriimino)-tris-benzoesäure-tris(2-ethylhexylester)
- 2,4-Bis-{[4-(2-Ethyl-hexyloxy)-2-hydroxy]-phenyl}-6-(4-methoxyphenyl)-1,3,5-triazin,
- 2,4-Bis-{[4-(3-sulfonato)-2-hydroxy-propyloxy)-2-hydroxy]-phenyl)-6-(4-methoxyphenyl)-1,3,5-triazin Natriumsalz,
- 2,4-Bis-{[4-(3-(2-Propyloxy)-2-hydroxy-propyloxy)-2-hydroxy]-phenyl}-6-(4-methoxyphenyl)-1,3,5-triazin,
- 2,4-Bis-{[4-(2-ethyl-hexyloxy)-2-hydroxy]-phenyl}-6-[4-(2-methoxyethyl-carboxyl)-phenylamino]-1,3,5-triazin,
- 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin,
- 2,4-Bis-{[4-(2-ethyl-hexyloxy)-2-hydroxy]-phenyl}-6-(1-methyl-pyrrol-2-yl)-1,3,5-triazin,
- 2,4-Bis-{[4-tris(trimethylsiloxy-silylpropyloxy)-2-hydroxy]-phenyl}-6-(4-methoxyphenyl)-1,3,5-triazin,
- 2,4-Bis-{[4-(2"-methylpropenyloxy)-2-hydroxy]-phenyl}-6-(4-methoxyphenyl)-1,3,5-triazin,
- 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Die Liste der genannten UV-Filter, die im Sinne der vorliegenden Erfindung eingesetzt werden können, soll selbstverständlich nicht limitierend sein.

Die Gesamtmenge an öllöslichen UV-Filtersubstanzen in den fertigen kosmetischen oder dermatologischen Zubereitungen, beispielsweise an 4,4',4"-(1,3,5-Triazin-2,4,6-triyltriimino)-tris-benzoesäure-tris(2-ethylhexylester) und/oder 4-(tert.-Butyl)-4'-meth-oxydibenzoylmethan und/oder 4-Methylbenzylidencampher, wird vorteilhaft aus dem Bereich von 0,1 - 20,0 Gew.-%, bevorzugt 0,5 - 10,0 Gew.-% gewählt, bezogen auf das Gesamtgewicht der Zubereitungen, falls die Gegenwart dieser Substanzen erwünscht ist.

Die Gesamtmenge an 2-Ethylhexyl-p-methoxy-cinnamat in den fertigen kosmetischen oder dermatologischen Zubereitungen wird, falls die Gegenwart dieser Substanz erwünscht ist, vorteilhaft aus dem Bereich von 0,1 - 15,0 Gew.-%, bevorzugt 0,5 - 7,5 Gew.-% gewählt, bezogen auf das Gesamtgewicht der Zubereitungen.

Die Gesamtmenge an Ethylhexyl-2-cyano-3,3-diphenylacrylat in den fertigen kosmetischen oder dermatologischen Zubereitungen wird, falls die Gegenwart dieser Substanz erwünscht ist, vorteilhaft aus dem Bereich von 0,1 - 15,0 Gew.-%, bevorzugt 0,5 - 10,0 Gew.-% gewählt, bezogen auf das Gesamtgewicht der Zubereitungen.

Die Gesamtmenge an einem oder mehreren Salicylsäurederivaten in den fertigen kosmetischen oder dermatologischen Zubereitungen wird vorteilhaft aus dem Bereich von 0,1 - 15,0 Gew.-%, bevorzugt 0,5 - 8,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 - 5,0 Gew.-%, bevorzugt 0,5 - 2,5 Gew.-% zu wählen. Wenn Homomenthylsalicylat gewählt wird, ist es von Vorteil, dessen Gesamtmenge aus dem Bereich von 0,1 - 10,0 Gew.-%, bevorzugt 0,5 - 5,0 Gew.-% zu wählen.

Kosmetische und dermatologische Formulierungen im Sinne der Erfindung enthalten einen oder mehrere übliche UV-A-, UV-B- und/oder Breitbandfilter als Einzelsubstanzen oder in beliebigen Gemischen untereinander, in der Lipidphase und/oder in der wäßrigen Phase.

Die Gesamtmenge an UV-Filtersubstanzen (UV-A-, UV-B- und/oder Breitbandfilter) in den fertigen kosmetischen oder dermatologischen Zubereitungen, sei es als Einzelsubstanz oder in beliebigen Gemischen untereinander, wird vorteilhaft aus dem Bereich von 0,1 bis 30 Gew.-%, bevorzugt 0,1 bis 20,0 Gew.-%, insbesondere 0,5 bis 15,0 Gew.-% gewählt, bezogen auf das Gesamtgewicht der Zubereitungen.

Erfindungsgemäße kosmetische und dermatologische Zubereitungen enthalten ferner vorteilhaft, wenngleich nicht zwingend, anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden. Diese Pigmente sind röntgenamorph oder nicht-röntgenamorph. Besonders bevorzugt handelt es sich um Pigmente auf der Basis von TiO₂.

Röntgenamorphe Oxidpigmente sind Metalloxide oder Halbmetalloxide, welche bei Röntgenbeugungsexperimenten keine oder keine erkenntliche Kristallstruktur erkennen lassen. Oftmals sind solche Pigmente durch Flammenreaktion erhältlich, beispielsweise dadurch, daß ein Metall- oder Halbmetallhalogenid mit Wasserstoff und Luft (oder reinem Sauerstoff) in einer Flamme umgesetzt wird.

In kosmetischen, dermatologischen oder pharmazeutischen Formulierungen werden röntgenamorphe Oxidpigmente als Verdickungs- und Thixotropierungsmittel, Fließhilfsmittel, zur Emulsions- und Dispersionsstabilisierung und als Trägersubstanz (beispielsweise zur Volumenerhöhung von feinteiligen Pulvern oder Pudern) eingesetzt.

Bekannte und in der kosmetischen oder dermatologischen Galenik oftmals verwendete röntgenamorphe Oxidpigmente sind die Siliciumoxide des Typs Aerosil^{®} (CAS-Nr. 7631-86-9. Aerosile^{®}, erhältlich von der Gesellschaft DEGUSSA, zeichnen sich durch geringe Partikelgröße (z.B. zwischen 5 und 40 nm) aus, wobei die Partikel als kugelförmige Teilchen sehr einheitlicher Abmessung anzusehen sind. Makroskopisch sind Aerosile^{®} als lockere, weiße Pulver erkenntlich. Im Sinne der vorliegenden Erfindung sind röntgenamorphe Siliciumdioxidpigmente besonders vorteilhaft, und unter diesen gerade solche des Aerosil^{®}-Typs bevorzugt.

Vorteilhafte Aerosil^{®}-Typen sind beispielsweise Aerosil^{®} OX50, Aerosil^{®} 130, Aerosil^{®} 150, Aerosil^{®} 200, Aerosil^{®} 300, Aerosil^{®} 380, Aerosil^{®} MOX 80, Aerosil^{®} MOX 170, Aerosil^{®} COK 84, Aerosil^{®} R 202, Aerosil® R 805, Aerosil^{®} R 812, Aerosil^{®} R 972, Aerosil^{®} R 974, Aerosil^{®} R976.

Erfindungsgemäß enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% röntgenamorphe Oxidpigmente.

Die nicht-röntgenamorphen anorganischen Pigmente liegen erfindungsgemäß vorteilhaft in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin daß die hydrophobe Oberflächenschicht nach einer Reaktion gemäß

n TiO₂ + m (RO)₃Si-R' → n TiO₂ (oberfl.)

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte TiO₂-Pigmente sind beispielsweise unter der Handelsbezeichnung T 805, vorteilhafte TiO₂/Fe₂O₃-Mischoxide unter der Handelsbezeichnung T 817 von der Firma Degussa erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die erfindungsgemäßen kosmetischen und/oder dermatologischen Formulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen. Entsprechend können die erfindungsgemäßen Zubereitungen, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zubereitungen als Grundlage für pharmazeutische Formulierungen zu verwenden. Bevorzugt sind insbesondere solche kosmetischen und dermatologischen Zubereitungen, die in der Form eines Hautpflege- bzw. Schminkproduktes vorliegen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich zu erfindungsgemäß verwendeten UV-A-, UV-B- und/oder Breitbandfiltern mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Die jeweils einzusetzenden Mengen an kosmetischen oder dermatologischen Hilfsund Trägerstoffen und Parfüm können in Abhängigkeit von der Art des jeweiligen Produktes vom Fachmann durch einfaches Ausprobieren leicht ermittelt werden.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 0,1 - 5 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:
- Mineralöle, Mineralwachse
- Öle,
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

## Patentansprüche

1. Kosmetische oder dermatologische Lichtschutzzubereitungen mit einem Gehalt an Bornitrid und UV-Filtersubstanzen, welche das Strukturmotiv des Triazins aufweisen, mit Ausnahme solcher Zubereitungen,
- welche höchstens 0,5 Gew.-% eines oder mehrerer Emulgatoren enthalten, und mit Ausnahme solcher Zubereitungen,
- welche mindestens eine polare Ölkomponente enthalten.

2. Lichtschutzzubereitungen nach Anspruch 1, **dadurch gekennzeichnet, daß** die Lichtschutzzubereitungen 0,1 bis 20 Gew.-%, vorteilhaft 0,5 bis 10 Gew.-%, ganz besonders bevorzugt 1 bis 5 Gew.-% Bornitrid enthalten.

3. Lichtschutzzubereitungen nach Anspruch 1 oder 2, **dadurch gekennzeichnet, daß** die Lichtschutzzubereitungen 0,1 bis 20 Gew.-%, vorteilhaft 0,5 bis 10 Gew.-%, an UV-Filtersubstanzen, welche das Strukturmotiv des Triazins aufweisen enthalten.

4. Lichtschutzzubereitungen nach einem der Ansprüche 1-3, **dadurch gekennzeichnet, daß** das oder die Triazinderivate gewählt werden aus der Gruppe
- 4,4',4' -(1,3,5-Triazin-2,4,6-triyltriimino)-tris-benzoesäure-tris(2-ethylhexylester)
- 2,4-Bis-{[4-(2-Ethyl-hexyloxy)-2-hydroxy]-phenyl}-6-(4-methoxyphenyl)-1,3,5-triazin,
- 2,4-Bis-{[4-(3-sulfonato)-2-hydroxy-propyloxy)-2-hydroxy]-phenyl}-6-(4-methoxyphenyl)-1,3,5-triazin Natriumsalz,
- 2,4-Bis-{[4-(3-(2-Propyloxy)-2-hydroxy-propyloxy)-2-hydroxy]-phenyl}-6-(4-methoxyphenyl)-1,3,5-triazin,
- 2,4-Bis-{[4-(2-ethyl-hexyloxy)-2-hydroxy]-phenyl}-6-[4-(2-methoxyethyl-carboxyl)-phenylamino]-1,3,5-triazin,
- 2,4-Bis-{[4-(3-(2-propyloxy)-2-hydroxy-propyloxy)-2-hydroxy]-phenyl)-6-[4-(2-ethyl-carboxyl)-phenylamino]-1,3,5-triazin,
- 2,4-Bis-{[4-(2-ethyl-hexyloxy)-2-hydroxy]-phenyl}-6-(1-methyl-pyrrol-2-yl)-1,3,5-triazin,
- 2,4-Bis-{[4-tris(trimethylsiloxy-silylpropyloxy)-2-hydroxy]-phenyl}-6-(4-methoxyphenyl)-1,3,5-triazin,
- 2,4-Bis-{[4-(2"-methylpropenyloxy)-2-hydroxy]-phenyl}-6-(4-methoxyphenyl)-1,3,5-triazin,
- 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

## Claims

1. Cosmetic or dermatological sunscreen preparations containing boron nitride and UV filter substances which have the structural motif of triazine, with the exception of those preparations
- which comprise at most 0.5% by weight of one or more emulsifiers,
and with the exception of those preparations,
- which comprise at least one polar oil component.

2. Sunscreen preparations according to Claim 1, **characterized in that** the sunscreen preparations comprise 0.1 to 20% by weight, advantageously 0.5 to 10% by weight, very particularly preferably 1 to 5% by weight, of boron nitride.

3. Sunscreen preparations according to Claim 1 or 2, **characterized in that** the sunscreen preparations comprise 0.1 to 20% by weight, advantageously 0.5 to 10% by weight, of UV filter substances which have the structural motif of triazine.

4. Sunscreen preparations according to one of Claims 1 - 3, **characterized in that** the triazine derivative or derivatives are selected from the group
- tris(2-ethylhexyl) 4,4',4'-(1,3,5-triazine-2,4,6-triyltriimino)trisbenzoate
- 2,4-bis{[4-(2-ethylhexyloxy)-2-hydroxy]phenyl}-6-(4-methoxyphenyl)-1,3,5-triazine,
- 2,4-bis{[4-(3-sulphonato)-2-hydroxypropyloxy)-2-hydroxy]phenyl}-6-(4-methoxyphenyl)-1,3,5-triazine sodium salt,
- 2,4-bis{[4-(3-(2-propyloxy)-2-hydroxypropyloxy)-2-hydroxy]phenyl}-6-(4-methoxyphenyl)-1,3,5-triazine,
- 2,4-bis{[4-(2-ethylhexyloxy)-2-hydroxy]phenyl}-6-[4-(2-methoxyethylcarboxyl)phenylamino]-1,3,5-triazine,
- 2,4-bis{[4-(3-(2-propyloxy)-2-hydroxypropyloxy)-2-hydroxy]phenyl}-6-[4-(2-ethylcarboxyl)phenylamino]-1,3,5-triazine,
- 2,4-bis{[4-(2-ethylhexyloxy)-2-hydroxy]phenyl}-6-(1-methylpyrrol-2-yl)-1,3,5-triazine,
- 2,4-bis{[4-tris(trimethylsiloxysilylpropyloxy)-2-hydroxy]phenyl}-6-(4-methoxyphenyl)-1,3,5-triazine,
- 2,4-bis{[4-(2"-methylpropenyloxy)-2-hydroxy]phenyl}-6-(4-methoxyphenyl)-1,3,5-triazine,
- 2,4-bis{[4-(1',1',1',3',5',5',5'-heptamethylsiloxy-2"-methylpropyloxy)-2-hydroxy]phenyl}-6-(4-methoxyphenyl)-1,3,5-triazine.

## Revendications

1. Préparations photoprotectrices cosmétiques ou dermatologiques contenant du nitrure de bore et des substances filtrant les UV, qui présentent le motif structural de la triazine, à l'exception des préparations
- qui contiennent au plus 0,5 % en poids d'un ou plusieurs émulsifiants,
et à l'exception des préparations
- qui contiennent au moins un composant huileux polaire.

2. Formulations photoprotectrices selon la revendication 1, **caractérisées en ce que** les préparations photoprotectrices contiennent 0,1 à 20 % en poids, avantageusement 0,5 à 10 % en poids, d'une manière tout particulièrement préférée 1 à 5 % en poids de nitrure de bore.

3. Préparations photoprotectrices selon la revendication 1 ou 2, **caractérisées en ce que** les préparations photoprotectrices contiennent 0,1 à 20 % en poids, avantageusement 0,5 à 10 % en poids, de substances filtrant les UV, qui présentent le motif structural de la triazine.

4. Préparations photoprotectrices selon l'une des revendications 1 à 3, **caractérisées en ce que** le ou les dérivés de la triazine sont choisis dans le groupe comprenant les composés suivants :
- l'ester tris(2-éthylhexylique) de l'acide 4,4',4"-(1,3,5-triazine-2,4,6-triyltriimino)-trisbenzoïque
- la 2,4-bis{[4-(2-éthyl-hexyloxy)-2-hydroxy]-phényl}-6-(4-méthoxyphényl)-1,3,5-triazine
- le sel de sodium de la 2,4-bis{[4-(3-sulfonato)-2-hydroxy-propyloxy)-2-hydroxy]-phényl}-6-(4-méthoxyphényl)-1,3,5-triazine
- la 2,4-bis{[4-(3-(2-propyloxy)-2-hydroxy-propyloxy)-2-hydroxy]-phényl}-6-(4-méthoxyphényl)-1,3,5-triazine
- la 2,4-bis{[4-(2-éthyl-hexyloxy)-2-hydroxy]-phényl}-6-[4-(2-méthoxyéthyl-carboxyl)-phényl-amino]-1,3,5-triazine
- la 2,4-bis-{[4-(3-(2-propyloxy)-2-hydroxy-propyloxy)-2-hydroxy]-phényl}-6-[4-(2-éthyl-carboxyl)-phénylamino]-1,3,5-triazine
- la 2,4-bis{[4-(2-éthyl-hexyloxy)-2-hydroxy]-phényl}-6-(1-méthyl-pyrrol-2-yl)-1,3,5-triazine
- la 2,4-bis{[4-tris(triméthylsiloxy-silylpropyloxy)-2-hydroxy]-phényl}-6-(4-méthoxyphényl)-1,3,5-triazine
- la 2,4-bis{[4-(2"-méthylpropényloxy)-2-hydroxy]-phényl}-6-(4-méthoxyphényl)-1,3,5-triazine
- la 2,4-bis{[4-(1',1',1',3',5',5',5'-heptaméthyl-siloxy-2"-méthyl-propyloxy)-2-hydroxy]-phényl}-6-(4-méthoxyphényl)-1,3,5-triazine.
